# EUROPEAN PATENT SPECIFICATION

(11) **EP 1 903 961 B1**
(45) Date of publication and mention of the grant of the patent: **16.05.2012**
(21) Application number: 06786685.5
(22) Date of filing: 07.07.2006
(51) Int. Cl.: A61B 17/58, A61B 17/34

(54) **DEVICE FOR DELIVERY OF BONE VOID FILLING MATERIALS**
VORRICHTUNG ZUR ABGABE VON FÜLLSTOFFEN FÜR KNOCHENLÜCKEN
DISPOSITIF D'ADMINISTRATION DE MATERIAUX DE REMPLISSAGE DE VIDE OSSEUX

(30) Priority: 07.07.2005 US 697146 P
(43) Date of publication of application: 02.04.2008
(73) Proprietor: Crosstrees Medical, Inc., Boulder, CO 80301 (US)
(72) Inventor: SCRIBNER, Robert, M., Longmont, CO 80503 (US); JONES, Lawrence, R., Conifer, CO 80433 (US)
(74) Representative: Johnstone, Douglas Ian
(86) International application number: PCT/US2006/026616
(87) International publication number: WO 2007/008721

(56) References cited:
- WO-A1-03/033363
- WO-A2-02/17801
- WO-A2-03/024369
- GB-A- 1 510 428
- US-A- 2 669 230
- US-A- 3 782 380
- US-A- 4 188 949
- US-A- 5 512 054
- US-A1- 2003 036 762
- US-A1- 2005 111 300

## Description

### Field of the Invention

The invention generally relates to the treatment of bones and/or other anatomical regions in humans and other animals.

### Background of the Invention

The creation of voids within bones and/or other body regions, including the deployment of expanding structures (i.e., balloons) or bone cutting instruments (i.e., drills and/or osteotomes) to create such voids, is well known in the art. As part of a fracture fixation procedure or other surgical intervention, a filling and/or stabilizing material, such as bone cement or other therapeutic compound, may desirably be introduced into a targeted void (or other location) within bone or other body regions to stabilize, support, repair and/or augment the targeted anatomical structure. Several companies offer bone cement injection devices. These devices are similar to a household caulking gun. Typically, the injection device has a pistol-shaped body, which supports a cartridge containing bone cement. The cement is typically in two or more parts (i.e., components) and must be mixed in a mixer and transferred into the cartridge for injection (or is mixed in the cartridge itself prior to or during injection). Just after mixing, and prior to curing, the filling/stabilization material is in a flowing, viscous liquid state, similar to a syrup or watery pancake batter in consistency. The injection device generally incorporates a ram or other flow inducing device, which is actuated by a manually movable trigger/screwing mechanism to push the viscous filling/stabilization material out the front of the cartridge through a suitable nozzle and into the interior of a bone targeted for treatment. Once injected into the targeted bone, the filling/stabilization material undergoes a curing cycle of perhaps six to eight minutes. While curing, the filling/stabilization material passes from a viscous liquid to a putty-like consistency and finally to a hard rigid block.

WO 02/17801, on which the preamble of claim 1 is based, discloses a filler instrument for introducing a desired amount of filling material into a bone. A two-stage material reservoir of the instrument comprises first and second sections. A first plunger is positioned within the first section, and a seal is secured to a distal end of the first plunger. A second plunger extends through a lumen of the first plunger

US 5512054 discloses a syringe for inflating a balloon catheter with liquid. A two-stage reservoir of the syringe comprises first and second sections. A first plunger is positioned within the first section, and a seal is secured to the first plunger. A second plunger extends through a lumen of the first plunger

US 2005/0111300 discloses a device having a mixing chamber forming a reservoir with a discharge aperture at one end. The chamber contains a discharge plunger having a portion extending beyond the opposite end of the chamber. A perforated mixing plunger is positioned in the mixing chamber between the discharge end and the discharge plunger and an actuating rod connected to the mixing plunger passes through the discharge plunger and extends beyond the mixing chamber. The mixing chamber is mounted within a hollow cylinder rigidly connected to the portion of the discharge plunger extending beyond the mixing chamber. External threads on the outside of mixing chamber engage internal threads on the inside of the hollow cylinder.

It is an object of the present invention to provide greater control over the placement of filling/stabilization material and/or other flowable liquids/materials into bone and/or other body regions.

### Summary of the Invention

In accordance with the present invention there is provided an introduction device for controlled injection of desired amounts of filling material into an anatomic structure, comprising a two-stage plunger coupled to a two-stage material reservoir in which the two-stage material reservoir comprises a first section and a second section, each section having a hollow tubular body sealingly connected to the other such that an interior of the first section is in fluid communication with an interior of the second section; the two-stage plunger comprising a first plunger and a second plunger, the first plunger being positioned within the first section, the first plunger being sized and configured to pass through an interior of the first section; the second plunger extending through a lumen of the first plunger when the first plunger is positioned within the first section, the second plunger being sized and configured to pass through an interior of the second section; and a seal secured to a distal end of the first plunger; characterized in that
the first section has internal screw threads, and the first plunger has external screw threads configured to engage the internal screw threads of the first section; and
the first and second plungers are rotatable together to longitudinally advance the first plunger through the first section and expel the filling material in the first section through the second section and out of a dispensing opening.

Embodiments of the invention may provide greater control over the placement of filling/stabilization material (such as PMMA bone cement) and/or other flowable liquids/materials into bone and/or other body regions, including the ability to introduce filler and/or stabilizing materials in a plurality of controlled manners without requiring removal/replacement of the injection device during the procedure. Embodiments may also facilitate the injection of highly viscous filling/stabilizing material into the bone or other tissues, either into a cavity formed within the bone or other tissues, or directly into the bone or other tissues themselves.

Features and advantages of various embodiments of the invention are set forth in the following Description and Drawings, as well as in the appended Claims.

### Brief Description of the Drawings

Fig. 1 is a lateral view of a human spinal column;
Fig. 2 is a top plan view, with portions broken away, of a human vertebral body;
Fig. 3 is a lateral view, with portions broken away of a series of vertebral bodies;
Fig. 4 is a perspective view of one embodiment of an introduction device constructed in accordance with various teachings of the invention;
Fig. 5 is another perspective view of the introduction device of Fig. 4;
Fig. 6 is a cut-away side view of the introduction device of Fig. 4;
Fig. 7 is a partial cut-away side view of the introduction device of Fig. 4;
Fig. 8 is another partial cut-away side view of the introduction device of Fig. 4.

The invention may be embodied in several forms without departing from its essential characteristics. The scope of the invention is defined in the appended claims, rather than in the specific description preceding them. All embodiments that fall within the meaning and range of equivalency of the claims are therefore intended to be embraced by the claims.

### Detailed Description of the Preferred Embodiments

This Specification describes new systems to facilitate the introduction of filling, stabilizing and/or therapeutic materials to treat bones and other anatomical structures. It should also be appreciated that the new systems can be utilized to treat bones without use of any cavity-creation and/or fracture reduction devices, if desired. The new systems will be described with regard to the treatment of vertebral bodies. It should be appreciated, however, that the systems so described are not limited in their application to vertebrae. The systems are applicable to the treatment of diverse bone types, including, but not limited to, such bones as the radius, the humerus, the femur, the tibia or the calcaneus, as well as other anatomical regions of human or animal bodies.

### Vertebral Bodies

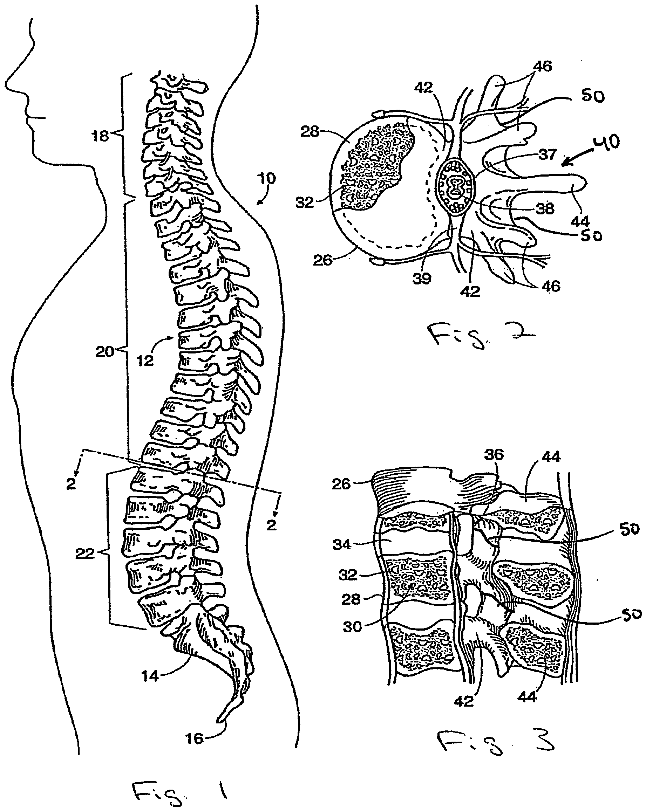

As Fig. 1 shows, the spinal column **10** comprises a number of uniquely shaped bones, called the vertebrae **12**, a sacrum **14**, and a coccyx **16** (also called the tail bone). The number of vertebrae **12** that make up the spinal column **10** depends upon the species of animal. In a human (which Fig. 1 shows), there are twenty-flour vertebrae **12**, comprising seven cervical vertebrae **18**, twelve thoracic vertebrae **20**, and five lumbar vertebrae **22**. When viewed from the side, as Fig. 1 shows, the spinal column **10** forms an S-shaped curve. The curve serves to support the head, which is heavy. In four-footed animals, the curve of the spine is simpler.

As Figs. 1 to 3 show, each vertebra **12** includes a vertebral body **26**, which extends on the anterior (*i.e*., front or chest) side of the vertebra **12**. The vertebral body **26** is in the shape of an oval disk. The vertebral body **26** includes an exterior formed from compact cortical bone **28**. The cortical bone **28** encloses an interior volume **30** of reticulated cancellous, or spongy, bone **32** (also called medullary bone or trabecular bone). A "cushion," called an intervertebral disk **34**, is located between the vertebral bodies **26**.

An opening, called the vertebral foramen **36**, is located on the posterior - *i.e*., back) side of each vertebra **12**. The spinal ganglion **39** pass through the foramen **36**. The spinal cord **38** passes through the spinal canal **37**. The vertebral arch **40** surrounds the spinal canal **37**. The pedicle **42** of the vertebral arch **40** adjoins the vertebral body **26**. The spinous process **44** extends from the posterior of the vertebral arch **40**, as do the left and right transverse processes **46**.

### Treatment of Vertebral Bodies

### Access to Targeted Anatomical Regions

Access to a vertebral body (or any other targeted anatomical regions) can be accomplished from many different directions, depending upon the targeted location within the vertebral body, the intervening anatomy, and the desired complexity of the procedure. For example, access can also be obtained through one or more pedicles (transpedicular), outside of a pedicle (extrapedicular), along either side of the vertebral body (posterolateral), superiorly or inferiorly (such as through an upper or lower intervertebral disc and/or adjacent vertebral bodies), laterally and/or anteriorly. In addition, such approaches can be used with a closed, minimally invasive procedure or with an open procedure (or some combination thereof).

### Instruments for Establishing Bone Access and Passage Creation

During a typical bilateral procedure to access a targeted vertebral body, a patient lies on an operating table. The patient can lie face down on the table, or on either side, or at an oblique angle, depending upon the physician's preference. The physician initially introduces a spinal needle assembly into soft tissue in the patient's back. Under radiologic or CT monitoring, the physician advances the spinal needle assembly through soft tissue down to and into the targeted vertebral body. The physician can also employ stereotactic instrumentation to guide advancement of the spinal needle assembly and subsequent tools during the procedure. In this arrangement, the reference probe for stereotactic guidance can be inserted through soft tissue and implanted on the surface of the targeted vertebral body. The entire procedure can also be monitored using tools and tags made of non-ferrous materials, e.g., plastic or fiber composites, such as those disclosed in U.S. Patents 5,782,764 and 5,744,958, which would be suitable for use in a computer enhanced, whole-room MRI environment.

The physician will typically administer a local anesthetic, for example, lidocaine, through the spinal needle assembly. In some cases, the physician may prefer other forms of anesthesia. The physician directs the spinal needle assembly to penetrate the cortical bone and/or cancellous bone through the side of the vertebral body. Preferably the depth of penetration is about 60% to 95% of the vertebral body. The physician holds the stylus and withdraws the stylet of the spinal needle assembly. The physician then slides a guide pin instrument through the stylus and into the cancellous bone. The physician now removes the stylus, leaving the guide pin instrument deployed within the cancellous bone.

The physician next slides an obturator instrument over the guide pin instrument, distal end first. The physician can couple the obturator instrument to a handle, which facilitates manipulation of the instrument. The physician makes a small incision in the patient's back. The physician twists the handle while applying longitudinal force to the handle. In response, the obturator instrument rotates and/or penetrates soft tissue through the incision. The physician may also gently tap the handle, or otherwise apply appropriate additional longitudinal force to the handle, to advance the obturator instrument through the soft tissue along the guide pin instrument down to the cortical bone entry site. The physician can also tap the handle with an appropriate striking tool to advance the obturator instrument into a side of the vertebral body to secure its position.

The obturator instrument has an outside diameter that is generally well suited for establishing a lateral access. However, if access is desired through a narrower region of the vertebral body, e.g., a pedicle (generally called a transpedicular access), the outside diameter of the obturator instrument can be reduced to well below the diameter of the pedicle. The reduced diameter of the obturator instrument mediates against damage or breakage of the pedicle. It should be understood that the disclosed devices are well suited for use in conjunction with other approach paths, such as pedicular, extra-pedicular, posterolateral and anterior approaches, with varying results. The physician then proceeds to slide the handle off the obturator instrument and to slide a cannula instrument over the guide pin instrument and, further, over the obturator instrument. If desired, the physician can also couple a handle to the cannula instruments, to apply appropriate twisting and longitudinal forces to rotate and advance the cannula instrument through soft tissue over the obturator instrument. When the cannula instrument contacts cortical bone, the physician can appropriately tap the handle with a striking tool to advance the end surface of the cannula into the vertebral body to secure its position.

The physician now withdraws the obturator instrument, leaving the cannula instrument in place. When a reduced or tapered diameter obturator instrument is used, the physician can remove an inner centering sleeve. The physician slides a drill bit instrument through the cannula instrument, until contact between the machined or cutting edge of the drill bit instrument and cortical bone occurs. The physician then couples the drill bit instrument to the handle. Guided by X-ray (or another external/non-invasive and/or partially invasive visualizing system), the physician applies appropriate twisting and longitudinal forces to the handle, to rotate and advance the machined edge of the drill bit instrument to open a lateral passage through the cortical bone and into the cancellous bone. The drilled passage preferably extends no more than 95% across the vertebral body.

### Additional Passage/Osteotomy Plane Creation

If desired, once access has been established, the physician may desire to create additional passages and/or one or more osteotomy planes within the targeted anatomical structure. Such additional passage/osteotomy plane(s) may be created within the bone and/or targeted anatomical structure(s) utilizing expandable structures (*i.e*., balloons) and/or bone cutting or manipulating instruments that can desirably be passed through one or more cannula instrument(s). Various devices and methods of using such devices is described in copending application entitled "Devices and Methods for the Treatment of Bone Fracture," filed concurrently.

### Introduction of Filling/Stabilizing/Therapeutic Materials

Once the passage, any additional passage(s) and/or one or more osteotomy planes have been created in the vertebral body (or if no such passage is formed or deemed necessary, as desired by the physician), the physician can introduce a filling/stabilizing and/or therapeutic material into the targeted vertebral body (or other anatomical location). The material can desirably comprise a material that resists torsional, tensile, shear and/or compressive forces within the passage, thereby providing renewed interior structural support for the surrounding cancellous and/or cortical bone, stabilize, secure and/or encapsulate cancellous and/or cortical bone fragments and/or serve as a source of therapeutic materials to treat medical conditions within and/or outside of the treated anatomical location. For example, the material can comprise a flowable material, such as bone cement, allograft tissue, autograft tissue, hydroxyapatite or other natural or synthetic bone substitute, which can be introduced into the passage and which, in time, sets to a more-hardened (desirably load-bearing and/or more stabilized) condition. The material can also comprise a compression-resistant material, such as rubber, polyurethane, cyanoacrylate, or silicone rubber, which is inserted into the passage. The material can also comprise a semi-solid slurry material (*e.g*., a bone slurry in a saline base), which is either contained within a porous fabric structure located in the passage or injected directly into the passage, to resist compressive forces within the passage. Alternatively, the material could comprise stents, reinforcing bar ("Re-Bar") or other types of internal support structures, which desirably resist compressive, tensile, torsional and/or shear forces acting on the bone and/or filler/stabilizing/therapeutic material. The filling/stabilizing material may also comprise medication, or a combination of medication and compression-resistant material, as described above.

Alternatively, the filling/stabilizing material can comprise a bone filling/stabilizing material which does not withstand compressive, tensile, torsional and/or shear forces within the cavity, or which does not immediately stabilize one or more of the surrounding bone fragments. For example, where the patient is not expected to experience significant forces within the spine immediately after surgery (*i.e.,* the patient is confined to bed rest or wears a brace), the filling/stabilizing material need not be able to immediately bear loads. Rather, the filling/stabilizing material could provide a scaffold for bone growth, or could comprise a material which facilitates or accelerates bone growth, allowing the bone to heal over a period of time. As another alternative, the filling/stabilizing material could comprise a resorbable or partially-resorbable source of organic or inorganic material for treatment of various bone or non-bone-related disorders including, but not limited to, osteoporosis, cancer, degenerative disk disease, heart disease, acquired immune deficiency syndrome (AIDS) or diabetes. In this way, the cavity and/or filler/stabilizing material could comprise a source of material for treatment of disorders located outside the treated bone.

### Material Containment Vessel

In one alternative embodiment, the physician may choose to utilize the invention in combination with a material containment vessel, such as the vessel disclosed in the copending application entitled "Devices and Methods for the Treatment of Bone Fracture," filed concurrently.

Following injection of the filling/stabilizing material, the vessel may be removed from the targeted bone, or can be left in the cavity, as desired by the physician. In such an arrangement, the filling/stabilizing material would be dispensed from the introduction device into the vessel, which desirably serves to contain the filling/stabilizing material and/or isolate the filling/stabilizing material from surrounding tissues for a period of time. Alternatively, the vessel, filled with the filling/stabilizing material, could serve to provide interior structural support function for the surrounding cancellous and/or cortical bone (if removal of the vessel is not desired by the physician). In various embodiments, the vessel could comprise an inert, durable, non-degradable plastic material, *e.g*., polyethylene and/or other polymers. Alternatively, the vessel could comprise an inert, bio-absorbable material, which degrades over time for absorption or removal by the body.

Desirably, the filling/stabilizing material or one of its components can additionally serve as an expansion medium for the vessel, allowing for the compaction of cancellous bone and/or formation of a passage, cavity and/or one or more osteotomy planes, and eventually perform compaction, interior support and/or stabilization functions. In an alternative embodiment, the vessel can be first expanded/enlarged with an expansion medium (*i.e*., to create a passage, cavity and/or osteotomy plane within the targeted anatomical region), and the filling/stabilization material can be subsequently introduced (either after the expansion material is removed or, if the expansion material forms a component of the filling/stabilizing material, concurrent with and/or after the expansion medium is introduced). In one alternative embodiment, the filling/stabilizing material could comprise a two-part material including, but not limited to, settable polymers or calcium alginate.

The vessel can comprise a permeable, semi-permeable, or porous material, which allows the transfer of medication or other materials through the wall of the vessel. If desired, the vessel can comprise a membrane that allows osmotic and/or particulate transfer through the vessel, or the vessel can comprise a material that allows the medication to absorb into and/or diffuse through the vessel. Alternatively, medication can be transported through a porous wall material by creating a pressure differential across the wall of the vessel, or fluids, cells and/or other materials from the patient's body can pass and/or be drawn through the vessel wall and into the vessel for various purposes including, but not limited to, fluid/cellular analysis, bony ingrowth, bone marrow harvesting, and/or gene therapy (including gene replacement therapy).

### Injection and/or Introduction of Materials

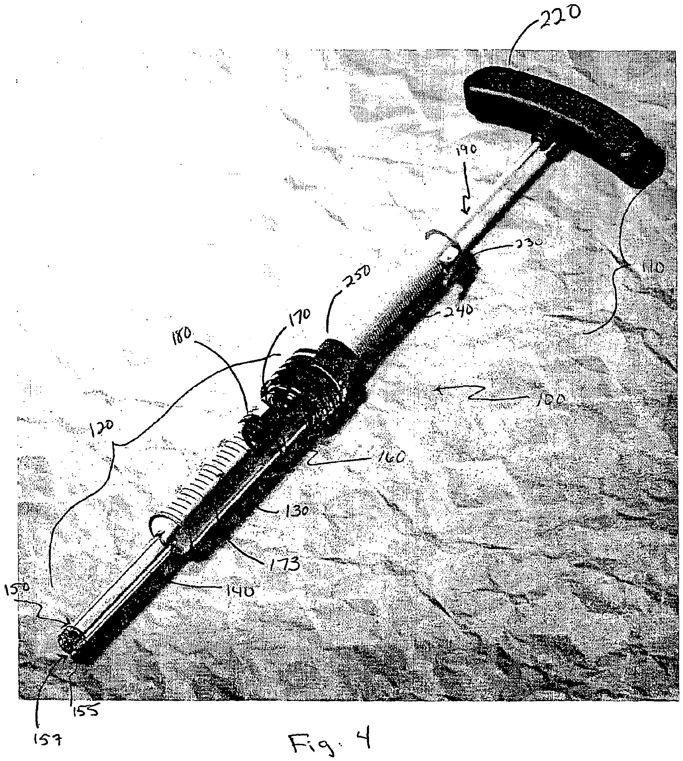

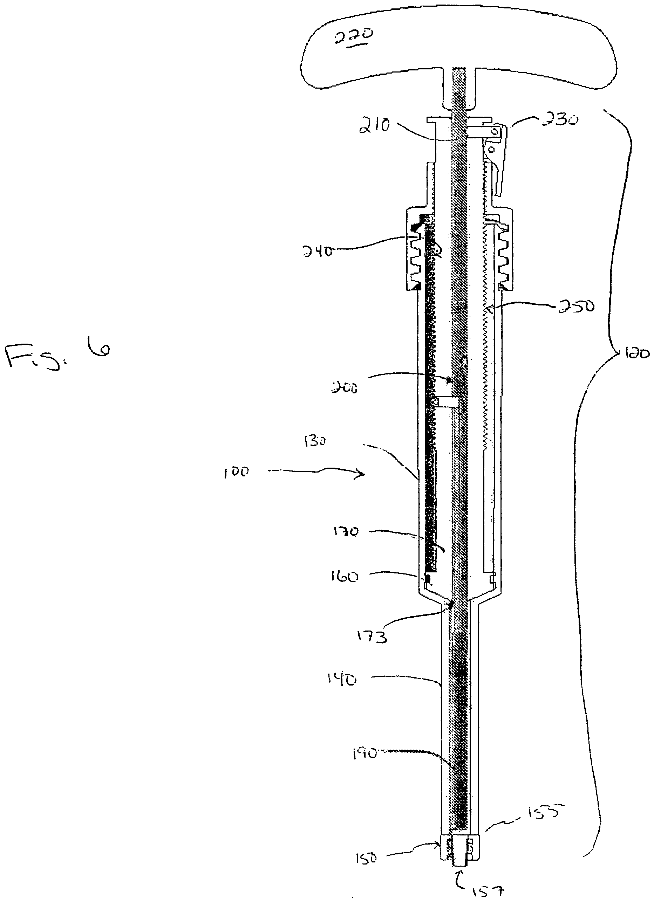

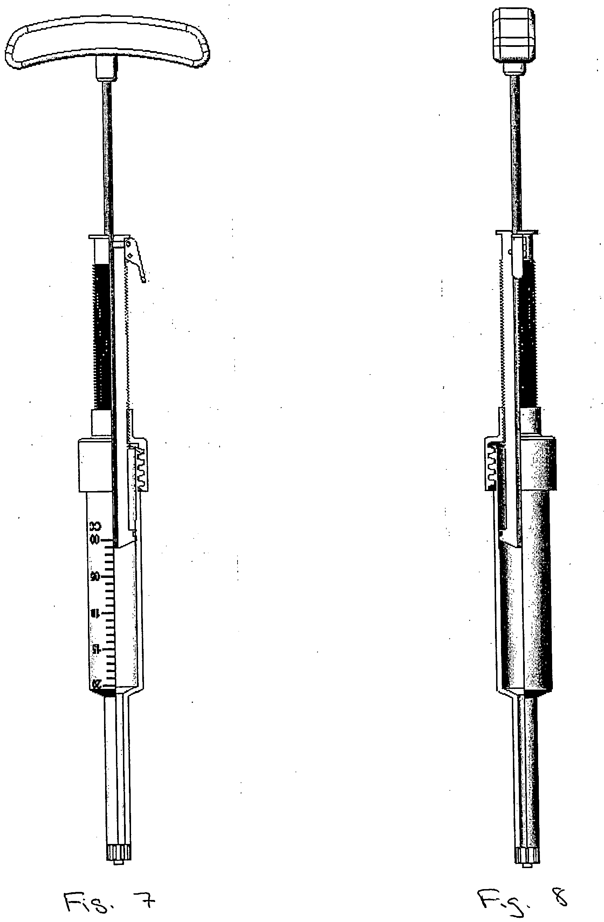

Figures 4-8 depict one embodiment of a material introduction device constructed in accordance with various teachings of the invention. Desirably, this device facilitates the controlled injection of desired amounts of filling/stabilizing material into a bone or other vertebral body (including into a vessel or other device contained therein). The introduction device **100** comprises a two-stage plunger **110** coupled to a two-stage material reservoir **120**. The two-stage reservoir **120** comprises a first section **130** and a second section **140**. The first and second sections **130** and **140** are hollow tubular bodies connected and/or secured in a sealing relationship, with the interior of the first section **130** being in fluid communication with the interior of the second section **140**. The first section **130** has a first interior cross-sectional area, and the second section **140** has a second interior cross-sectional area. Desirably, the first interior cross-sectional area will be greater than the second interior cross-sectional area. If also desired, the transition from the first section **130** to the second section **140** can neck down or taper.

In the disclosed embodiment, the first section **130** comprises a cylindrical, hollow, tubular member having an interior diameter and a length as determined by the application, and the second section **140** comprises a cylindrical, hollow, tubular member having an interior diameter and a length as determined by the application. A luer fitting **150** (or other suitable connection device) is positioned around a dispensing opening **157** at the distal tip **155** of the second section **140**. In one embodiment, the luer fitting **150** is desirably sized and configured to dock or mate with a corresponding fitting (not shown) on a corresponding catheter attached to a material containment vessel (not shown). Of course, the distal tip **155** could alternatively incorporate a dispensing opening **157** without a luer fitting **150**, or possibly an atraumatic dispensing tip or similar docking mechanism, if desired. Alternatively, the distal tip can be designed to have a conical or tapered end for easy advancement and immediate use for injecting directly into bone or other targeted anatomical structure(s). The distal tip may also be designed to have radiopaque markers for easy positioning and placement during use of indirect/non-invasive and/or minimally invasive visualization techniques known in the art.

The two-stage plunger **110** comprises a first ram or plunger **170**, positioned within the first section **130**, which is sized and configured to pass through the interior of the first section **130**. A seal **160**, such as an O-ring, is secured to the distal end **180** of the first plunger **170** in a manner well known in the art. Desirably, the seal **160** will slidingly engage with the inner walls of the first section **130** to seal the proximal end of the first section **130** as the first plunger **170** advances therethrough. The seal **160** can comprise polytetrafluoroethylene (PTFE, also known under the tradename of "Teflon"), natural rubber, or other type of known sealant material. The seal can also be coated with a hydrophilic or hydrophobic material that facilitates easy translation through the first section **130**. It should be noted that, while the cross-section of the disclosed plunger appears circular in Fig. 4, plungers having other cross-sectional shapes, such as triangular or rectangular shapes, could similarly be utilized. An opening **173** is formed in the central area of the seal **160**.

If desired, the plunger and cylinder wall materials can be designed to withstand the linear forces and/or temperatures of the exothermal or chemical reagents of other biochemical reaction(s) of the mixed filler/stabilizing material without bending, breaking or otherwise significantly degrading, such as hard plastics or metals. The inner diameter of the cylinder(s) can also be coated with the same hydrophilic or hydrophobic material to allow easy translation of the seal. The external body of the first plunger can include or have printed graduations for measurements or amount of material injected as commonly used in the art.

The two-stage plunger 110 further comprises a second ram or plunger 190, at least a portion of which is sized and configured to extend through a lumen 200 formed within the first plunger **170** (as well as in the opening **173** formed in the seal **160**). A T-handle **220** or other suitable manipulating device is positioned at a proximal end of the second plunger **190**. In another embodiment, the T-handle **220** and remainder of the device can be designed to have a lumen accommodating a guidewire for easy positioning and/or placement and/or removal of the guidewire through the device. The second plunger **190** desirably extends through the lumen **200** such that the distal end of the second plunger sits approximately flush with the opening **173** in the seal **160**. One or more securement or latching mechanisms **230** are positioned on the first plunger **170**, desirably engaging a corresponding detent or notch **210** or other engagement mechanism on the second plunger **190**. When engaged, the latching mechanism **230** secures the first and second plungers **170** and **190** together, but when disengaged, the second plunger **190** may be advanced longitudinally through the lumen **200** of the first plunger **170**. If desired, the shaft of the second plunger **190** and the corresponding lumen **200** need not be circular or cylindrical in shape, but rather may be a range of shapes that prevent and/or inhibit relative rotation between the lumen **200** and the second plunger **190**, yet desirably allow longitudinal movement of the second plunger **190** when the latching mechanism **230** is released.

Desirably, the shape and size of the cross-sectional area of the second plunger **190** will approximate the shape and size of the cross-sectional area of the second section 140, such that the second plunger is capable of displacing substantially all of the filler/stabilizing material in the second section as the second plunger advances. Similarly, the shape and size of the cross-sectional area of the first plunger **170** (or that of the seal **160**) will desirably approximate the shape and size of the cross-sectional area of the first section **130**, such that the first plunger is capable of displacing substantially all of the filler/stabilizing material in the first section as the first plunger advances. In an alternative embodiment, the second plunger can be designed to accommodate a guidewire for easy positioning and/or placement of the device. For material strength, the various components of the introduction device **100** can comprise a substantially rigid metal, plastic or ceramic material (*i.e*., stainless steel or a high strength plastic) or some combination thereof.

The first plunger **170** further incorporates a series of external screw threads **240**, which engage with corresponding internal screw threads **250** within the first section **130**. Desirably, the corresponding sets of screw threads **240** and **250** are sized and configured such that, in the disclosed embodiment, one complete rotation of the first plunger would result in the expellation of approximately 1 cc of material contained within the first section **130**. Of course, other thread designs and arrangements could be utilized to allow other defined controlled injection amounts. The threads desirably provide a mechanical "force multiplication" advantage, converting the rotation of the T-handle and plungers to a longitudinal advancement of the first plunger within the first section and a commensurate dispensing of filler/stabilizing material. The first cylinder surrounding the first plunger can also be designed to be interchangeable or dispensable in case filler/stabilizing material hardens and/or cures - allowing a new cylinder to be introduced to replace one containing hardened filler/stabilizing material and used accordingly. Alternatively, the body of the first plunger may also incorporate a one-way valve or seal that prevents the retrograde flow of blood and/or other body fluids, yet still allows translation of the second plunger **190** through the extended nose or body of the second section **140**. If desired, a side port or relief valve can be integrated into the device to release any trapped air that may be contained within the filler/stabilization material or materials that may embolize and enter the device during injection of the filler/stabilizing material. The air port could accommodate a seal, one-way valve or cap to cover and/or eliminate expellation and/or introduction of air or other gases and/or fluids.

When injection of filler/stabilizing material is desired, the first and/or second sections **130** and/or **140** of the introduction instrument **100** can be filled with filler/stabilizing material (not shown) such as bone cement or PMMA. As the T-handle is rotated in a clockwise direction, the first and second plungers **170** and **190** rotate together, advancing the first plunger **170** through the first section **130** and expelling filler/stabilizing material in the first section **130** through the second section **140** and out the dispensing opening **157**. Passage of a significant amount of filler/stabilizing material back through the opening **173** in the central area of the seal **160** is desirably prevented by the presence of the second plunger **190** in the opening **173.**

When a desired amount of filling/stabilizing material has been expelled, or thickening/polymerization of the filler/stabilizing material has rendered the material within the device so resistant to flow that rotation of the T-handle is no longer safe or feasible, it may still be desirous to expel an additional metered amount of filler/stabilizing material to ensure the clinical objectives. In such a case, the illustrated embodiment allows the physician to disengage the latching mechanism **230**, and the second plunger **190** may then be pushed into and through the first section **130**, desirably expelling an additional amount of filler/stabilizing material equal to the volume of the second plunger entering the first section **130**. Further advancement of the second plunger **190** in this manner will allow the second plunger to eventually enter and displace filler/stabilizing material within the second section **140**, which in the disclosed embodiment would desirably amount to an additional 1-1.5 cc of filler/stabilizing material.

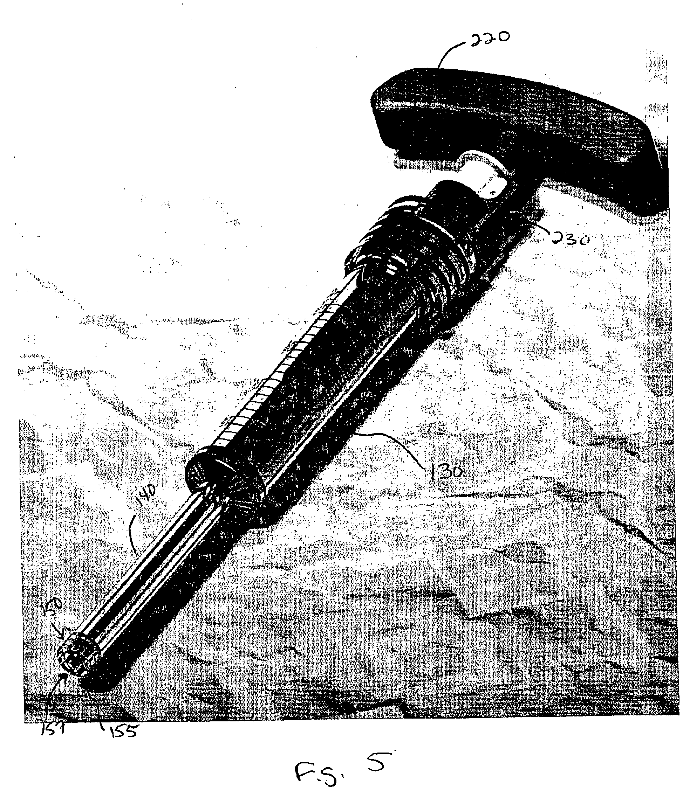

If desired, the second plunger **190** may be of a length sufficient to travel to the end of the dispensing opening **157** (see Fig. 5). Alternatively, the second plunger **190** could be of a significantly longer length, potentially allowing the distal tip of the second plunger **190** to travel through and past the dispensing opening **157**, ultimately into the surgical instruments used to access the targeted anatomical structures (*i.e*., catheter tubing and/or minimally-invasive cannulae) and/or into the anatomical structures themselves. In such an arrangement, the second plunger could potentially be utilized to tamp and evacuate any residual filler/stabilizing material still within the cannula, and/or manipulate filling/stabilizing material within the targeted anatomical region.

If desired, the second plunger may incorporate additional detents along its length such that, during advancement of the second plunger, the latching mechanism causes an audible or tactile "click" to indicate the approximate position of the advancing second plunger relative to the latching mechanism, or that can temporarily "lock" the latching mechanism to prevent accidental withdrawal of the second plunger.

By utilizing first and second sections of different cross-sectional areas, and first and second plungers to displace the filler/stabilizing material, the illustrated embodiment facilitates dispensing of a substantial amount of filler/stabilizing material from a single introduction device. Because the viscosity of PMMA and various other types of filler/stabilizing materials typically increases with time during the dispensing process, it becomes progressively harder to dispense filler/stabilizing material over time. By utilizing a plunger of larger cross-sectional area to initiate the filling operation, when the filler/stabilizing material is less viscous, the illustrated embodiment allows dispensing of a significant amount of filler/stabilizing material through rotation of the T-handle. However, as the filler/stabilizing material cures, and becomes more viscous, the reduced cross-sectional area of the second plunger allows continued dispensing of the more viscous filler/stabilizing material, even when it is in a highly viscous state. Moreover, because the second section is of reduced cross-sectional area, its reduced profile will desirably allow the distal tip of the filler instrument to be introduced through a cannula and/or soft tissues and directly into a targeted vertebral body, while still providing a sufficient reservoir of filler/stabilizing material to accomplish the goals of augmenting/stabilizing and/or repairing the targeted bone.

Moreover, because the introduction device need not be refilled and/or "switched out" during the dispensing operation, and can remain in place and dispense the entire required amount of bone filler/stabilizing material for the procedure, the potential for trapping air within the vertebral body and/or bolus of filler/stabilizing material is significantly reduced. The illustrated embodiment also greatly facilitates the ability of the physician to immediately shift from a higher volume, lower pressure filler/stabilizing material flow to a lower volume, higher pressure filler/stabilizing material flow. As the first plunger is being advanced, and filler/stabilizing material is being injected into the vertebral body, the physician may determine that a more controlled, higher pressure and/or lower volume flow of filler/stabilizing material is needed. Alternatively, the filler/stabilizing material may cure or harden to a point where further movement of the first plunger is extremely difficult and/or impossible to effect. One embodiment of the invention permits the physician to advance the second plunger into the second section, even when the distal end of the first plunger is not adjacent, near and/or abutting the distal end of the first section. As the second plunger passes through the filler/stabilizing material in the first section, and enters the second section, filler/stabilizing material will desirably continue to be displaced from the dispensing opening. Due to the decreased cross-sectional area of the second plunger and second section (as compared to the first plunger and first section), the second plunger may more easily be pushed through the filling/stabilizing material in the first section and filling/stabilizing material can more easily be dispensed from the second section at higher pressures and/or lower volumes.

The disclosed introduction device may be used to introduce filler/stabilizing material through a cannula into a cavity created within a bone, or may be used with vertebroplasty-type techniques to introduce filler/stabilizing material directly into the vertebral body without prior formation of a cavity. Where prior cavity-formation is not required and/or desired, and vertebroplasty-like techniques will be used, the filler instrument can incorporate a needle-point at the distal end of the instrument, or the diameter of the second section can be significantly reduced to allow passage of the instrument (or the second plunger, if desired) through the lumen of a spinal needle assembly.

Alternatively, one or more of the sections of the introduction device could comprise a commercially available spinal needle assembly (such as a Bone Marrow Biopsy Needle, available from Becton Dickinson & Co., Franklin Lakes, NJ, 07417 USA). If desired, one or more plunger assemblies of varying sizes and lengths could be provided to accommodate differing spinal needle assemblies.

If desired, the introduction device can be pre-loaded with filler/stabilizing material, introduced through soft tissues and into the targeted vertebral body, used to inject filler/stabilizing material, and removed, quickly and easily without need for tool exchanges during the operation. For example, where the end plates of the vertebral body have depressed to a point where a cavity cannot be safely created within the vertebral body *(i.e.,* through the use of bone manipulating instruments and/or expandable structures), filling/stabilizing material can be introduced under pressure through a needle directly into the cancellous bone of the vertebral body. The filler/stabilizing material desirably penetrates cancellous bone.

The delivery of bone void filling/stabilizing materials to anatomic locations can be accomplished by direct placement to the void site in an open surgical setting, or by percutaneous delivery to the void site by means of delivery devices. The size and shape of any devices disclosed herein are desirably selected by the physician, taking into account the morphology and/or geometry of the site to be treated. The shape of the joint, the bones and soft tissues involved, and the local structures that could be harmed if moved inappropriately, are generally understood by medical professionals using textbooks of human anatomy along with their knowledge of the site and its disease and/or injury. The physician is also desirably able to select the desired shape and/or size of instrument(s) and its/their placement in and/or around the joint (or other anatomical structure) based upon prior analysis of the morphology of the targeted anatomical region using, for example, plain film x-ray, fluoroscopic x-ray, MRI or CT scanning. The shape, size and placement are desirably selected to optimize the strength and ultimate bonding of the filling/stabilizing material to the surrounding bone and/or tissues of the targeted anatomical region.

The systems embodying the invention can be adapted for use virtually in any interior body region, include body regions where the formation of a cavity or void within tissue is or is not required and/or desired for a therapeutic or diagnostic purpose. The preferred embodiments show the invention in association with systems used to treat bones. This is because the systems which embody the invention are well suited for use in this environment.

It should be appreciated that the systems which embody features of the invention can be used in other interior body regions, including other non-bone body regions, which are also contemplated. It should also be understood that the principles of the various embodiments of the invention could be applied by those skilled in the art to a wide variety of mammals and/or other animals, with varying results.

Although the disclosure hereof is detailed and exact to enable those skilled in the art to practice the invention, the physical embodiments herein disclosed merely exemplify the invention that may be embodied in other specific structures. Other embodiments and uses of the invention will be apparent to those skilled in the art from consideration of the specification and practice of the invention disclosed herein. All documents referenced herein are specifically and entirely incorporated by reference. The specification and examples should be considered exemplary only with the true scope of the invention indicated by the following claims. As will be easily understood by those of ordinary skill in the art, variations and modifications of each of the disclosed embodiments, including combinations or the various embodiments disclosed herein, can be easily made within the scope of this invention as defined by the following claims.

## Claims

1. An introduction device (100) for controlled injection of desired amounts of filling material into an anatomic structure, comprising a two-stage plunger (110) coupled to a two-stage material reservoir (120) in which the two-stage material reservoir (100) comprises a first section (130) and a second section (140), each section (130, 140) having a hollow tubular body sealingly connected to the other such that an interior of the first section (130) is in fluid communication with an interior of the second section (140); the two-stage plunger (110) comprising a first plunger (170) and a second plunger (190), the first plunger (170) being positioned within the first section (130), the first plunger (170) being sized and configured to pass through an interior of the first section (130); the second plunger (190) extending through a lumen (200) of the first plunger (170) when the first plunger (170) is positioned within the first section (130), the second plunger (190) being sized and configured to pass through an interior of the second section (140); and a seal (160) secured to a distal end (180) of the first plunger (170); **characterized in that**
the first section (130) has internal screw threads (250), and the first plunger (170) has external screw threads (240) configured to engage the internal screw threads (250) of the first section (130); and
the first and second plungers (170, 190) are rotatable together to longitudinally advance the first plunger (170) through the first section (130) and expel the filling material in the first section (130) through the second section (140) and out of a dispensing opening (157).

2. The device of claim 1, in which the first section (130) has a first interior cross-sectional area, and the second section (140) has a second interior cross-sectional area, and the first interior cross-sectional area is greater than the second interior cross-sectional area.

3. The device of claim 1 or 2, wherein the dispensing opening (157) is at a distal tip (155) of the second section (140).

4. The device of claim 3, further comprising a connection fitting (150) positioned around the dispensing opening (157) at the distal tip (155) of the second section (140).

5. The device of claim 1 or 2, further comprising an atraumatic dispensing tip at a distal end (155) of the second section (140).

6. The device of claim 1 or 2, further comprising a conical distal tip of the second section (140) that is configured for direct injection into the anatomic structure.

7. The device of any preceding claim, in which the seal (160) slidingly engages with the interior of the first section (130) to seal the proximal end of the first section (130) as the first plunger (170) advances therethrough.

8. The device of any preceding claim, in which the cross-section of the second plunger (190) is one which appears circular, triangular or rectangular.

9. The device of any preceding claim, further comprising a lumen adapted for at least one of positioning, placement, and removal of a guidewire through the device (100).

10. The device of any preceding claim, in which the second plunger (190) extends through the lumen (200) of the first plunger (170) such that the distal end of the second plunger (190) is approximately flush with an opening (173) in the seal (160).

11. The device of any one of claims 1 to 9, in which the second plunger (190) is disposed within an opening (173) in the central area of the seal (160) such that the second plunger (190) substantially prevents any filling material from passing back through the opening (173) in the seal (160).

12. The device of any preceding claim, further comprising at least one securement mechanism (230) positioned on the first plunger (170) and engaging a corresponding engagement mechanism (210) on the second plunger (190) to secure the first and second plungers (170, 190) together, the at least one securement mechanism (230) configured to permit the second plunger (190) to be advanced longitudinally through the lumen (200) of the first plunger (170) when disengaged from the corresponding engagement mechanism (210) on the second plunger (190).

13. The device of claim 12, in which the second plunger (190) and the lumen (200) of the first plunger (170) are shaped in cross-section to inhibit relative rotation between the lumen (200) of the first plunger (170) and the second plunger (190), and to allow longitudinal movement of the second plunger (190) when the engagement mechanism (210) is released from the at least one securement mechanism (230).

14. The device of any one of claims 1 to 11, including a latching mechanism, and in which the second plunger (190) includes at least one detent along its length for the latching mechanism to engage in a manner to indicate the approximate position of the second plunger (190) relative to the latching mechanism during advancement of the second plunger (190).

15. The device of any one of claims 1 to 11, including a latching mechanism, and in which the second plunger (190) includes at least one detent along its length for locking the latching mechanism so as to prevent accidental withdrawal of the second plunger (190) during advancement of the second plunger (190).

16. The device of any preceding claim, in which the shape and size of the cross-sectional area of the second plunger (190) approximate the shape and size of the cross-sectional area of the second section (140) such that the second plunger (190) is capable of displacing substantially all of the filling material in the second section (140) as the second plunger (190) advances through the second section (140).

17. The device of any preceding claim, in which the shape and size of the cross-sectional area of at least one of the first plunger (170) and the seal (160) approximate the shape and size of the cross-sectional area of the first section (130) such that the first plunger (170) is capable of displacing substantially all of the filling material in the first section (130) as the first plunger (170) advances through the first section (130).

18. The device of any preceding claim, in which the second plunger (190) accommodates a guidewire for at least one of positioning and placement of the device (100).

19. The device of any preceding claim, in which the internal screw threads (250) of the first section (130) and the external screw threads (240) of the first plunger (170) are sized and configured such that one complete rotation of the first plunger (170) expels approximately 1 cc of filling material contained within the first section (130).

20. The device of any preceding claim, in which the internal screw threads (250) of the first section (130) and the external screw threads (240) of the first plunger (170) convert rotation of a T-handle (220) and the first and second plungers (170, 190) to longitudinal advancement of the first plunger (170) within the first section (130) and a commensurate dispensing of filling material.

21. The device of any preceding claim, in which the first plunger (170) includes a one-way valve to prevent retrograde flow of body fluids without preventing translation of the second plunger (190) through the interior of the second section (140).

22. The device of any preceding claim, further comprising a relief valve to release at least one of air contained within the filling material and other material that may enter the device (100) during injection of the filling material.

23. The device of any preceding claim, in which when the second plunger (190) is pushed through the first section (130), the second plunger (190) is configured to expel an amount of filling material equal to the volume of the second plunger (190) entering the first section (130).

24. The device of any preceding claim, in which the second plunger (190) has a length sufficient to travel to the end of the dispensing opening (157).

25. The device of any one of claims 1 to 23, in which the second plunger (190) has a length sufficient to allow the distal tip of the second plunger (190) to travel through and past the dispensing opening (157).

## Patentansprüche

1. Einführungsvorrichtung (100) zur kontrollierten Injektion einer gewünschten Menge eines Füllmaterials in eine anatomische Struktur, umfassend einen mit einem zweistufigen Materialreservoir (120) gekoppelten zweistufigen Kolben (110), wobei das zweistufige Materialreservoir (100) einen ersten Abschnitt (130) und einem zweiten Ab schnitt (140) umfasst, wobei jeder Abschnitt (130, 140) einen derart mit dem anderen abdichtend verbundenen hohlen, rohrförmigen Körper hat, dass ein Innenraum des ersten Abschnitts (130) in Fluidverbindung mit einem Innenraum des zweiten Abschnitts (140) steht; der zweistufige Kolben (110) umfasst einen ersten Kolben (170) und einen zweiten Kolben (190), wobei der erste Kolben (170) in dem ersten Abschnitt (130) positioniert ist, und der erste Kolben (170) so in der Größe angepasst und ausgebildet ist, um durch einen Innenraum des ersten Abschnitts (130) hindurchzugehen; der zweiten Kolben (190) erstreckt sich durch ein Lumen (200) des ersten Kolbens (170), wenn der erste Kolben (170) innerhalb des ersten Abschnitts (130) positioniert ist, wobei der zweite Kolben (190) so in der Größe angepasst und ausgebildet ist, um durch einen Innenraum des zweiten Abschnitts (140) hindurchzugehen; und eine Dichtung (160), welche an einem Distalende (180) des ersten Kolbens (170) befestigt ist, **dadurch gekennzeichnet, dass**
der erste Abschnitt (130) ein Innengewinde (250) aufweist, und der erste Kolben (170) ein Außengewinde (240) aufweist, das zum Eingriff mit dem Innengewinde (250) des ersten Abschnitts (130) ausgebildet ist; und
der erste und zweite Kolben (170,190) miteinander rotierbar sind, um den ersten Kolben (170) durch den ersten Abschnitt (130) längs voranzubewegen und das in dem ersten Abschnitt (130) befindliche Füllmaterial durch den zweiten Abschnitt (140) und aus einer Ausgabeöffnung (157) auszubringen.

2. Vorrichtung nach Anspruch 1, wobei der erste Abschnitt (130) einen ersten Innenquerschnittsbereich hat und der zweite Abschnitt (140) einen zweiten Innenquerschnittsbereich hat, und der erste Innenquerschnittsbereich größer als der zweite Innenquerschnittsbereich ist.

3. Vorrichtung nach Anspruch 1 oder 2, wobei sich die Ausgabeöffnung (157) an einer distalen Spitze (155) des zweiten Abschnitts (140) befindet.

4. Vorrichtung nach Anspruch 3, weiter umfassend ein um die Ausgabeöffnung (157) an der distalen Spitze (155) des zweiten Abschnitts (140) positioniertes Verbindungsstück (150).

5. Vorrichtung nach Anspruch 1 oder 2, weiter umfassend eine atraumatische Ausgabespitze an einem Distalende (155) des zweiten Abschnitts (140).

6. Vorrichtung nach Anspruch 1 oder 2, weiter umfassend eine konische distale Spitze des zweiten Abschnitts (140), die zur direkten Injektion in die anatomische Struktur ausgebildet ist.

7. Vorrichtung nach einem der voranstehenden Ansprüche, wobei die Dichtung (160) gleitend mit dem Innenraum des ersten Abschnitts (130) in Eingriff kommt, um das Proximalende des ersten Abschnitts (130) abzudichten, wenn sich der erste Kolben (170) dadurch voranbewegt.

8. Vorrichtung nach einem der voranstehenden Ansprüche, wobei der Querschnitt des zweiten Kolbens (190) einer ist, der kreisförmig, dreiecksförmig oder rechteckförmig aussieht.

9. Vorrichtung nach einem der voranstehenden Ansprüche, weiter umfassend ein Lumen, dass zumindest für die Positionierung, Platzierung und/oder Entfernung eines Führungsdrahts durch die Vorrichtung (100) ausgebildet ist.

10. Vorrichtung nach einem der voranstehenden Ansprüche, wobei sich der zweite Kolben (190) derart durch das Lumen (200) des ersten Kolbens (170) erstreckt, dass das Distalende des zweiten Kolbens (190) in etwa bündig mit einer Öffnung (173) in der Dichtung (160) ist.

11. Vorrichtung nach einem der Ansprüche 1 bis 9, wobei der zweite Kolben (190) derart innerhalb einer Öffnung (173) im Zentralbereich der Dichtung (160) angeordnet ist, dass der zweite Kolben (190) im wesentlichen das Zurücklaufen von Füllmaterial durch die Öffnung (173) in der Dichtung (160) verhindert.

12. Vorrichtung nach einem der voranstehenden Ansprüche, weiter umfassend mindestens einen Sicherungsmechanismus (230), der an dem ersten Kolben (170) positioniert ist und einen korrespondierenden Eingriffsmechanismus (210) an dem zweiten Kolben (190) in Eingriff nimmt, um den ersten und zweiten Kolben (170, 190) aneinander zu sichern, wobei der mindestens eine Sicherungsmechanismus (230) ausgebildet ist, um es dem zweiten Kolben (190) zu ermöglichen, in Längsrichtung durch das Lumen (200) des ersten Kolbens (170) voranbewegt zu werden, wenn er vom korrespondierenden Eingriffsmechanismus (210) an dem zweiten Kolben (190) gelöst ist.

13. Vorrichtung nach Anspruch 12, wobei der zweite Kolben (190) und das Lumen (200) des ersten Kolbens (170) im Querschnitt derart geformt sind, um eine relative Rotation zwischen dem Lumen (200) des ersten Kolbens (170) und dem zweiten Kolben (190) zu verhindern, und eine Längsbewegung des zweiten Kolbens (190) zu ermöglichen, wenn der Eingriffsmechanismus (210) von dem mindestens einen Sicherungsmechanismus (230) gelöst ist.

14. Vorrichtung nach einem der Ansprüche 1 bis 11, umfassend einen Verriegelungsmechanismus, und wobei der zweite Kolben (190) entlang seiner Länge mindestens eine Rastung für den Verriegelungsmechanismus aufweist, um in einer Art eingreifen zu können, in der die ungefähre Position des zweiten Kolbens (190) relativ zum Verriegelungsmechanismus während der Voranbewegung des zweiten Kolbens (190) angezeigt wird.

15. Vorrichtung nach einem der Ansprüche 1 bis 11, umfassend einen Verriegelungsmechanismus, und wobei der zweite Kolben (190) entlang seiner Länge mindestens eine Rastung zum Verriegeln des Verriegelungsmechanismus aufweist, um ein unbeabsichtigtes Zurückziehen des zweiten Kolbens (190) während der Voranbewegung des zweiten Kolbens (190) zu verhindern.

16. Vorrichtung nach einem der voranstehenden Ansprüche, wobei die Form und Größe des Querschnittsbereichs des zweiten Kolbens (190) ungefähr der Form und Größe des Querschnittsbereichs des zweiten Abschnitts (140) entspricht, so dass der zweite Kolben (190) dazu geeignet ist, im wesentlichen das gesamte Füllmaterial im zweiten Abschnitt (140) auszubringen, wenn sich der zweite Kolben (190) durch den zweiten Abschnitt (140) voranbewegt.

17. Vorrichtung nach einem der voranstehenden Ansprüche, wobei die Form und Größe des Querschnittsbereichs zumindest des ersten Kolbens (170) und/oder der Dichtung (160) in etwa der Größe und Form des Querschnittsbereichs des ersten Abschnitts (130) entsprechen, so dass der erste Kolben (170) dazu geeignet ist, im wesentlichen das gesamte Füllmaterial im ersten Abschnitt (130) auszubringen, wenn sich der erste Kolben (170) durch den ersten Abschnitt (130) voranbewegt.

18. Vorrichtung nach einem der voranstehenden Ansprüche, wobei der zweite Kolben (190) einen Führungsdraht mindestens zum Positionieren und/oder Platzieren der Vorrichtung (100) aufnimmt.

19. Vorrichtung nach einem der voranstehenden Ansprüche, wobei das Innengewinde (250) der ersten Abschnitts (130) und das Außengewinde (240) des ersten Kolbens (170) so in der Größe angepasst und ausgebildet sind, dass eine vollständige Umdrehung des ersten Kolbens (170) ungefähr 1 cc des im ersten Abschnitt (130) enthaltenen Füllmaterials ausbringt.

20. Vorrichtung nach einem der voranstehenden Ansprüche, wobei das Innengewinde (250) des ersten Abschnitts (130) und das Außengewinde (240) des ersten Kolbens (170) die Rotation eines T-Griffs (220) und des ersten und zweiten Kolbens (170, 190) in eine longitudinale Voranbewegung des ersten Kolbens (170) innerhalb des ersten Abschnitts (130) und eine entsprechende Ausgabe von Füllmaterial umwandeln.

21. Vorrichtung nach einem der voranstehenden Ansprüche, wobei der erste Kolben (170) einen Einwegventil aufweist, um einen retrograden Fluss von Körperflüssigkeiten zu verhindern, ohne die Translation des zweiten Kolbens (190) durch den Innenraum des zweiten Abschnitts (140) zu behindern.

22. Vorrichtung nach einem der voranstehenden Ansprüche, weiter umfassend ein Ablaufventil zum Ablassen von innerhalb des Füllmaterials enthaltener Luft und/oder anderen Materialien, die während der Injektion des Füllmaterials in die Vorrichtung (100) eintreten.

23. Vorrichtung nach einem der voranstehenden Ansprüche, wobei, wenn der zweite Kolben (190) durch den ersten Abschnitt (130) gedrückt wird, der zweite Kolben (190) ausgebildet ist, um eine Menge an Füllmaterial auszugeben, die dem Volumen des in den ersten Abschnitt (130) eintretenden zweiten Kolbens (190) entspricht.

24. Vorrichtung nach einem der voranstehenden Ansprüche, wobei der zweite Kolben (190) eine ausreichende Länge hat, um zum Ende der Ausgabeöffnung (157) bewegt zu werden.

25. Vorrichtung nach einem der Ansprüche 1 bis 23, wobei der zweite Kolben (190) eine ausreichende Länge hat, um es der distalen Spitze des zweiten Kolbens (190) zu ermöglichen, durch und hinter die Ausgabeöffnung (157) bewegt zu werden.

## Revendications

1. Dispositif d'introduction (100) pour l'injection contrôlée de quantités souhaitées d'un matériau de remplissage dans une structure anatomique, qui comprend un plongeur à deux étages (110) couplé à un réservoir de matériau à deux étages (120) dans lequel le réservoir de matériau à deux étages (100) comprend une première section (130) et une seconde section (140), chaque section (130, 140) ayant un corps tubulaire creux hermétiquement connecté à l'autre de sorte qu'un intérieur de la première section (130) se trouve en communication fluidique avec un intérieur de la seconde section (140) ; le plongeur à deux étages (110) comprenant un premier plongeur (170) et un second plongeur (190), le premier plongeur (170) étant positionné à l'intérieur de la première section (130), le premier plongeur (170) ayant une taille et une configuration lui permettant de traverser un intérieur de la première section (130) ; le second plongeur (190) s'étendant à travers une lumière (200) du premier plongeur (170) lorsque le premier plongeur (170) est positionné à l'intérieur de la première section (130), le second plongeur (190) ayant une taille et une configuration lui permettant de traverser un intérieur de la seconde section (140) ; et un joint (160) fixé à une extrémité distale (180) du premier plongeur (170) ; **caractérisé en ce que**
la première section (130) a des filetages internes (250), et le premier plongeur (170) a des filetages externes (240) configurés pour se mettre en prise avec les filetages internes (250) de la première section (130) ; et
les premier et second plongeurs (170, 190) peuvent pivoter ensemble pour faire avancer longitudinalement le premier plongeur (170) à travers la première section (130) et expulser le matériau de remplissage de la première section (130) à travers la seconde section (140) et par une ouverture d'administration (157).

2. Dispositif selon la revendication 1, dans lequel la première section (130) a une première section transversale intérieure, et la seconde section (140) a une seconde section transversale intérieure, et la première section transversale intérieure est supérieure à la seconde section transversale intérieure.

3. Dispositif selon la revendication 1 ou 2, dans lequel l'ouverture d'administration (157) se trouve au niveau d'une extrémité distale (155) de la seconde section (140).

4. Dispositif selon la revendication 3, comprenant en outre un raccord de connexion (150) positionné autour de l'ouverture d'administration (157) au niveau de l'extrémité distale (155) de la seconde section (140).

5. Dispositif selon la revendication 1 ou 2, comprenant en outre une extrémité d'administration atraumatique au niveau d'une extrémité distale (155) de la seconde section (140).

6. Dispositif selon la revendication 1 ou 2, comprenant en outre une extrémité distale conique de la seconde section (140) qui est configurée pour l'injection directe dans la structure anatomique.

7. Dispositif selon l'une quelconque des revendications précédentes, dans lequel le joint (160) se met en prise par coulissement avec l'intérieur de la première section (130) pour fermer l'extrémité proximale de la première section (130) alors que le premier piston (170) progresse à travers celle-ci.

8. Dispositif selon l'une quelconque des revendications précédentes, dans lequel la section transversale du second plongeur (190) est circulaire, triangulaire ou rectangulaire.

9. Dispositif selon l'une quelconque des revendications précédentes, comprenant en outre une lumière adaptée pour au moins l'un du positionnement, du placement, et du retrait d'un fil-guide dans le dispositif (100).

10. Dispositif selon l'une quelconque des revendications précédentes, dans lequel le second plongeur (190) s'étend à travers la lumière (200) du premier plongeur (170) de manière à ce que l'extrémité distale du second plongeur (190) soit approximativement au même niveau qu'une ouverture (173) dans le joint (160).

11. Dispositif selon l'une quelconque des revendications 1 à 9, dans lequel le second plongeur (190) est disposé à l'intérieur d'une ouverture (173) dans la zone centrale du joint (160) de manière à ce que le second plongeur (190) empêche sensiblement le retour de tout matériau de remplissage à travers l'ouverture (173) dans le joint (160).

12. Dispositif selon l'une quelconque des revendications précédentes, comprenant en outre au moins un mécanisme de fixation (230) positionné sur le premier plongeur (170) et se mettant en prise avec un mécanisme de prise correspondant (210) sur le second plongeur (190) pour fixer les premier et second plongeurs (170, 190) ensemble, le ou les mécanismes de fixation (230) étant configurés pour permettre la progression longitudinale du second plongeur (190) à travers la lumière (200) du premier plongeur (170) lorsqu'il est séparé du mécanisme de prise correspondant (210) sur le second plongeur (190).

13. Dispositif selon la revendication 12, dans lequel le second plongeur (190) et la lumière (200) du premier plongeur (170) ont une section transversale façonnée pour inhiber la rotation relative entre la lumière (200) du premier plongeur (170) et le second plongeur (190), et pour permettre le mouvement longitudinal du second plongeur (190) lorsque le mécanisme de prise (210) est libéré du ou des mécanismes de fixation (230).

14. Dispositif selon l'une quelconque des revendications 1 à 11, qui comprend un mécanisme de verrouillage, et dans lequel le second plongeur (190) comprend au moins un encliquetage sur sa longueur pour permettre la mise en prise du mécanisme de verrouillage d'une manière qui indique la position approximative du second plongeur (190) relativement au mécanisme de verrouillage pendant la progression du second plongeur (190).

15. Dispositif selon l'une quelconque des revendications 1 à 11, comprenant un mécanisme de verrouillage et dans lequel le second plongeur (190) comprend au moins un encliquetage sur sa longueur pour bloquer le mécanisme de verrouillage afin de prévenir tout retrait accidentel du second plongeur (190) pendant la progression du second plongeur (190).

16. Dispositif selon l'une quelconque des revendications précédentes, dans lequel la forme et la taille de la section transversale du second plongeur (190) se rapprochent de la forme et la taille de la section transversale de la seconde section (140) de telle sorte que le second plongeur (190) est capable de déplacer sensiblement tout le matériau de remplissage dans la seconde section (140) alors que le second plongeur (190) progresse à travers la seconde section (140).

17. Dispositif selon l'une quelconque des revendications précédentes, dans lequel la forme et la taille de la section transversale d'au moins l'un du premier plongeur (170) et du joint (160) se rapprochent de la forme et la taille de la section transversale de la première section (130) de telle sorte que le premier plongeur (170) est capable de déplacer sensiblement tout le matériau de remplissage dans la première section (130) alors que le premier plongeur (170) progresse à travers la première section (130).

18. Dispositif selon l'une quelconque des revendications précédentes, dans lequel le second plongeur (190) renferme un fil-guide pour au moins l'un du positionnement et du placement du dispositif (100).

19. Dispositif selon l'une quelconque des revendications précédentes, dans lequel les filetages internes (250) de la première section (130) et les filetages externes (240) du premier plongeur (170) ont une taille et une configuration telles qu'une rotation complète du premier plongeur (170) entraîne l'expulsion d'approximativement 1 cm³ de matériau de remplissage contenu à l'intérieur de la première section (130).

20. Dispositif selon l'une quelconque des revendications précédentes, dans lequel les filetages internes (250) de la première section (130) et les filetages externes (240) du premier plongeur (170) convertissent la rotation d'une poignée en T (220) et des premier et second plongeurs (170, 190) en une progression longitudinale du premier plongeur (170) à l'intérieur de la première section (130) et une distribution proportionnelle du matériau de remplissage.

21. Dispositif selon l'une quelconque des revendications précédentes, dans lequel le premier plongeur (170) comprend une valve unidirectionnelle destinée à empêcher un écoulement rétrograde des fluides biologiques sans empêcher la translation du second plongeur (190) à travers l'intérieur de la seconde section (140).

22. Dispositif selon l'une quelconque des revendications précédentes, comprenant en outre un clapet de décharge pour libérer au moins l'un de l'air contenu à l'intérieur du matériau de remplissage et d'un autre matériau qui pourrait entrer dans le dispositif (100) durant l'injection du matériau de remplissage.

23. Dispositif selon l'une quelconque des revendications précédentes, dans lequel lorsque le second plongeur (190) est poussé durant la première injection (130), le second plongeur (190) est configuré pour expulser une quantité de matériau de remplissage égale au volume du second plongeur (190) entrant dans la première section (130).

24. Dispositif selon l'une quelconque des revendications précédentes, dans lequel le second plongeur (190) est suffisamment long pour aller jusqu'à l'extrémité de l'ouverture d'administration (157).

25. Dispositif selon l'une quelconque des revendications 1 à 23, dans lequel le second plongeur (190) est suffisamment long pour permettre que l'extrémité distale du second plongeur (190) aille jusqu'à et au-delà de l'ouverture d'administration (157).
